# EUROPEAN PATENT SPECIFICATION

(11) **EP 1 551 480 B1**
(45) Date of publication and mention of the grant of the patent: **13.05.2009**
(21) Application number: 03798092.7
(22) Date of filing: 26.09.2003
(51) Int. Cl.: A61M 5/14

(54) **INDICATING DEVICE WITH ESTIMATING FEATURE**
INDIKATORGERÄT MIT ABSCHÄTZUNGSMERKMALEN
DISPOSITIF INDICATEUR AVEC CARACTERISTIQUE D'ESTIMATION

(30) Priority: 30.09.2002 DK 200201448
(43) Date of publication of application: 13.07.2005
(73) Proprietor: NOVO NORDISK A/S, 2880 Bagsvaerd (DK)
(72) Inventor: HANSEN, Steffen, DK-3400 Hillerod (DK); KLITGAARD, Peter, Christian, DK-2765 Smorum (DK); HANSEN, Per, Hvid, DK-3450 Lynge (DK)
(86) International application number: PCT/DK2003/000631
(87) International publication number: WO 2004/028596

(56) References cited:
- EP-A- 0 681 847
- EP-A- 1 177 802
- WO-A-00/10628
- US-A- 4 146 029
- US-A- 4 898 578

## Description

### FIELD OF THE INVENTION

The present invention relates to an indicating device for conveniently providing a user of a portable drug delivery device with information in respect of the delivery process, as well as a system comprising a drug delivery system in combination with an indicating device.

A drug delivery system and an indicating device as defined in the preamble of claim 1 and 13, respectively, are disclosed in EP-A-1 177 802.

### BACKGROUND OF THE INVENTION

In the disclosure of the present invention reference is mostly made to the treatment of diabetes by infusion of insulin, however, this is only an exemplary use of the present invention.

Diabetes mellitus is the common name for at least 2 different diseases, one characterised by immune system mediated specific pancreatic beta cell destruction (insulin dependent diabetes mellitus (IDDM) or type 1 diabetes), and another characterised by decreased insulin sensitivity (insulin resistance) and/or a functional defect in beta cell function (non-insulin dependent diabetes mellitus (NIDDM) or type 2 diabetes).

The principal treatment of type 1 diabetes is straight forward substitution of the missing insulin secretion, whereas treatment of type 2 is more complicated. More specifically, in early stages of type 2 diabetes treatment a number of different types of drugs can be used, e.g. drugs which increase insulin sensitivity (ciglitazones), decrease hepatic glucose output (e.g. metformin), or reduce glucose uptake from the gut (alfa glucosidase inhibitors), as well as drugs which stimulate beta cell activity (e.g. sulfonylurea/meglitinides). However, the above-described deterioration is reflected in the fact that beta cell stimulators will eventually fail to stimulate the cell, and the patient has to be treated with insulin, either as mono therapy, or in combination with oral medication in order to improve glucose control.

Currently, there are two principal modes of daily insulin therapy, the first mode including syringes and insulin injection pens. These devices are simple to use and are relatively low in cost, but they require a needle stick at each injection, typically 3-4 times or more per day. The second mode is infusion pump therapy, which entails the purchase of a portable but relatively expensive pump, for which reason the initial cost of the pump is a barrier to this type of therapy. Although more complex than syringes and pens, the pump offer the advantages of continuous infusion of insulin, precision in dosing and optionally programmable delivery profiles and user actuated bolus infusions in connections with meals. Recently less expensive infusion pumps have been proposed which may either be fully disposable providing only the most basic functions such as a constant basal rate, or infusion pump systems comprising a disposable portion in combination with a durable control portion, where the latter may provide many of the more advanced features of the traditional pump.

Early portable pumps were often intended for implantation which consequently resulted in a barrier for user access to the pump. To provide the user with means for controlling such a pump, US patent 4,146,029 discloses an implantable insulin pump device which can have its settings modified by extracorporeal wireless control means (e.g. using a RF-transmitter) so as to provide a selectable, programming capability. The same object is addressed in US patent 4,559,037 which discloses a control device for wireless transmission of program instructions to an insulin pump unit which may be either implanted or external to the body. The control device may be used to select a desired basal rate, to select a given infusion schedule or to command the infusion of a bolus having a desired size and infusion profile. The disclosed control device may be programmable and may comprise a display. WO 00/10628 discloses a similar system in which a remote commander can be used to selectively activate a desired function in an external infusion pump device, e.g. delivery of a bolus, selecting a profile for the bolus, or selecting a basal infusion rate. The remote commander comprises a display allowing the user to visually confirm the values entered into the remote commander.

The above-described "remote" control devices are primarily adapted for command purposes, i.e. to transmit user actuated program instructions to an infusion device being either implanted or carried under the clothing. However, it has also been proposed to use a remote device in combination with an insulin infusion system for the purpose of providing the user with information associated with the performance of the infusion system. More specifically, US patent 5,569,186 discloses a closed loop infusion pump system comprising an infusion pump which responds to control signals from an implantable glucose sensor unit to deliver medication as needed to the patient. The glucose sensor is associated with a telemetry unit transmitting glucose measurement signal information by means of radio telemetry to the infusion pump for closed loop operation thereof. The glucose measurements may alternately be transmitted to an externally located monitor in the form of a wrist-worn device. The monitor can be operated in response to information received and/or displayed from either the pump or the glucose sensor to thereby control pump operation through the use of radio telemetry signals. The monitor may be programmed to automatically adjust pump operation according to glucose measurements (i.e. the monitor is an integral portion of the closed loop system), or to recommend a treatment program to allow patient verification and manual initiation, or to simply display the glucose readings and permit manual entry of reprogramming commands.

Similarly, EP 1 177 802 discloses a wearable self-contained drug infusion device which may be used in combination with a programmer in wireless communication therewith. The communication is preferably two-way which allows the programmer to transmit flow rate settings and other commands to the drug infusion device, and also allows the drug infusion device to transmit confirmation or these settings and commands back to the programmer. The return communication link also allows the drug infusion unit to transmit status information back to the programmer so that it can be displayed to the user on a display screen, e.g. the actual flow rate or insulin in the device.

As appears from the above, remote monitors (or commanders) have been proposed for use in combination with drug infusion devices, which provide the user with status information in respect of the infusion device or a glucose sensor. However, this means (i) that either the user have to actuate the remote monitor device to provide transmission of current status data from the infusion device each time information is required, or (ii) the remote monitor and the infusion device have to be in constant or regular contact with each other to provide updated information. Neither of these solutions appears to be optimal. From the users point of view it would be desirable to have access to the information just at a glance without having to actuate any buttons, like when looking swiftly at a wristwatch. From the manufacturers point of view constant updating of the data information in the monitor device would require a relatively strong and thereby energy-consuming transmitter, e.g. a RF transmitter, as an automatic update function should ensure that the monitor can be "reached" even under the difficult receiving conditions. However, even a strong infusion device transmitter would only be able to communicate with the monitor device when the two devices are within "normal" reach of each other, i.e. when the monitor is carried by the user. Thus, even the most reliable transmission would not be able to cope with the situation that the monitor is not constantly carried by the user, which means that the user could be provided with incorrect non-updated information when the monitor is picked up after a certain period of time.

### DISCLOSURE OF THE INVENTION

Having regard to the above-identified problems and deficiencies, it is an object of the present invention to provide an indicating device (or "monitor") in combination with or for use in combination with a delivery device, in which the user has ready access to the desired information and which can be manufactured in a cost-effective manner. The latter requirement would be an important aspect for a disposable or semi-disposable delivery device for which it normally would be too expensive to incorporate "long-distance constantly transmitting" transmitter means.

More specifically, the present invention is based on the concept that the desired information can be provided by estimating actual values on the basis of known data. More specifically, an indicating device which can be carried conveniently by the user is provided with information in respect of the actual delivery taking place, on the basis of which the desired values can be calculated.

Thus, in a first aspect the present invention provides a drug delivery system comprising a delivery device and an indicating device. The delivery device is adapted for delivering a drug from a reservoir into a body of a user in accordance with a delivery rate value or profile, and comprises means for providing data information (in the following also just data) to the indicating device. The indicating device comprises means for receiving and storing data information from the infusion device, as well as timer means, where (second) processor means is adapted for calculating an estimated time-dependent value based upon the received data and time information, and associated with indication means (e.g. a display) for indicating a calculated value. The means for providing data information may in a simple form be "passive" means which can be read or activated by the indicating device, e.g. a bar code printed on the delivery device or an induction-activated IC, however, in exemplary embodiments the delivery device comprises cooperating first processor and transmission means for transmitting data information to the indicating device. Indeed, is data transferred to the indicating device only when taking a new pump into use, then all subsequently displayed values will rely on a calculated estimate.

In an exemplary embodiment the first processor means is adapted for transmitting data representing an amount of drug currently contained in the reservoir (i.e. at the time when data is transmitted), and the second processor means is adapted for calculating a time-dependent estimate for the drug on the basis of the current amount, the time information, and an infusion rate value stored in the memory means. The infusion rate may be stored in the indicating device (e.g. as a preset value or input by a user) or the rate may be transmitted as data from the infusion device. On the basis of these basic data it is thus possible to calculate an estimate for the amount of the drug infused at a given time, typically the current time. In case data representing an initial amount of drug contained in the reservoir is transmitted (or preset), it will also be possible to calculate the amount of drug remaining in the reservoir. In an exemplary embodiment, if the remaining amount of drug is below a preset level an alarm signal will result (e.g. visual or audible). Instead of a constant rate, an infusion profile may be utilized, e.g. a lower or higher infusion rate may be used during pre-selected periods during the day such as during the night.

As the estimated values are based on the assumption that the actual infusion takes place in accordance with the set rate, it may be desirable to provide the user with information as to when the data on which the calculations are based was transmitted to the indication device, i.e. updated. For this purpose the second processor means may be adapted for determining the time lapsed since the last data information was received, and for indicating information indicative of the time lapsed. The latter may take place in different forms, e.g. the actual time of the last update may be shown in combination with the estimated value or it may be indicated when a certain amount of time has lapsed since the last update, e.g. by a flashing value.

Depending on the complexity of the respective devices, the update may be performed manually or automatically. For example, for a simple configuration for the indication device, updates may be transmitted from the delivery device either automatically (e.g. every 5 minutes, every hour or at any other given interval) and/or manually when the delivery device is actuated by the user. An automatic update function would normally imply that the transmission means (such as a RF transmitter) is capable of reaching the indication device when the latter is located within the intended range, e.g. when worn or carried by the patient. In case short-distance transmission means (such as IR or induction) is used, it will normally be necessary for the user to bring the indication device within the reach of the transmitter and then actuate the latter. In both cases, the above-described information as to the last update will be useful.

In a more complex configuration, the indication and delivery devices are adapted for two-way communication. In such a system the delivery device comprises receiving means for receiving commands transmitted from the indicator device, and the indicator device comprises transmission means for transmitting commands to the infusion device in response to user actuation or controlled by the second processor means, whereby data transmission updates from the delivery device can be initiated by commands from the indicating device. In an exemplary embodiment, the commands are sent at predetermined intervals, e.g. every 5 minutes or every hour or with any other desired interval. To save energy, signals which are "confirmed" may initially be sent with low intensity which then may be increased until proper communication has been established.

In an exemplary embodiment the indicating device further comprises user-actuatable means for inputting a bolus command, e.g. an additional amount of insulin typically taken at mealtime, where the second processor means is adapted for transmitting the bolus command to the delivery device, wherein the delivery device is adapted for delivering a bolus in response to the bolus command. In order to be sure that the bolus command is properly received, data may be returned to the indicating device confirming that the bolus command has been received, after which the second processor means can calculate time-dependent estimates for the drug utilizing the additional data information for the bolus command.

To protect against disturbances from the outside or interference between different systems of the same time operating in the vicinity of each other, the first processor means may be adapted to transmit ID data information indicative of a unique infusion device. When the system is initialized (e.g. when the pump is refilled or a new prefilled pump is taken into use), this ID will be stored in the memory of the indication device and the second processor means will check the ID data information before storing updated data information in the memory means. Initialization may be based on data transmission from the delivery device, typically when it is started, or on commands from the indicating device, e.g. the delivery device may be started by a start command from the indicating device resulting in start of delivery as well as return transmission of ID data information. In case the delivery device is started without the indicating device being within reach of the transmitted signals, the delivery device advantageously is adapted to be "re-started" to thereby transmit updated information to the indicating device.

The data stored in the memory means may also be displayed directly without serving as a basis for calculations. For example, for a given (short) period after update the displayed values may correspond to the actually transmitted values, or the user may recall this information at any given time. In this situation, the display means may indicate whether actual or calculated data is displayed. In an exemplary embodiment, the indication means is in the form of a display allowing continuous display of an estimated (or actual) value.

As appears from the above description, the two devices of the system are adapted to cooperate with each other, however, they need not to be supplied and/or marketed as a system. More specifically, an infusion device may be sold in which the transmitting/receiving capabilities are "hidden" for the user. The indication/command device may then be offered as an optional "remote" device in combination with which the "hidden" features of the infusion device can be activated or utilized. For example, when used alone the delivery device provides a basal delivery rate of a given drug whereas in combination with the indication/command device a bolus function can be activated.

Correspondingly, in a second aspect the present invention provides an indicating device comprising means for receiving data information, memory means for storing data information, timer means, processor means adapted for calculating an estimated time-dependent value based upon received data and time information, and indication means for indicating a calculated value. The means for receiving data information may adapted to cooperate with "passive" means which can be read or activated by the indicating device, e.g. a bar code printed on a delivery device or an induction-activated IC, however, in exemplary embodiments the indicating device comprises receiving means for receiving a remotely generated signal containing the data information.

In exemplary embodiments the indicating device may comprise any of the above-described features for an indicating device as well as being adapted for cooperation with the correspondingly described delivery device, i.e. capable of transmitting and receiving the described data information.

The present invention thus provides an indication device and system which provides the user with ready access to the desired information and which can be manufactured in a cost-effective manner. However, the estimating feature of the invention also allows the system to be used in alternative ways which would not be possible with the above-described known systems.

For example, a child who is using an infusion device for insulin delivery is going to the kindergarten or nursery school in the morning. Just before the attending parent leaves the child he/she gets the indicating device updated with the latest information from the infusion device. The parent is now able to follow the estimated amount of insulin left in the reservoir without being near the child and to get an alarm if the amount of insulin left in the reservoir is to low. Indeed, such a feature could also be realized using e.g. mobile phone technology, however, it readily appears that from a cost point of view, this would be expensive to implement.

In the above description of aspects of the invention a system comprising two units are described, however, the indication device and system of the invention may also be used in combination with further components. For example, the indication device may be adapted to communicate (transmit and/or receive) with one or more additional components, either by the same or by additional communication means, e.g. IR for short distance communication with a delivery device or other body-mounted devices such as a glucose sensor, and RF for longer distance communication with more remotely located components. The more remotely located components may e.g. be a local or distant computer system to which data is transmitted and/or received.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormone, biologically derived or active agents, hormonal and gene based agents, nutritional formulas and other substances in both solid (dispensed) or liquid form. In the description of the exemplary embodiments reference will be made to the use of insulin. Correspondingly, the term "subcutaneous infusion" is meant to encompass any method of parenteral delivery to a subject.

### BRIEF DESCRIPTION OF THE DRAWINGS

In the following the invention will be further described with references to the drawings, wherein
fig. 1 is a schematic representation of a first embodiment of the invention,
fig. 2 is a schematic representation of a second embodiment of the invention,
fig. 3 shows a third embodiment, and
fig. 4 shows a flow chart for a system corresponding to the third embodiment.

In the figures like structures are identified by like reference numerals.

### DESCRIPTION OF EXEMPLARY EMBODIMENTS

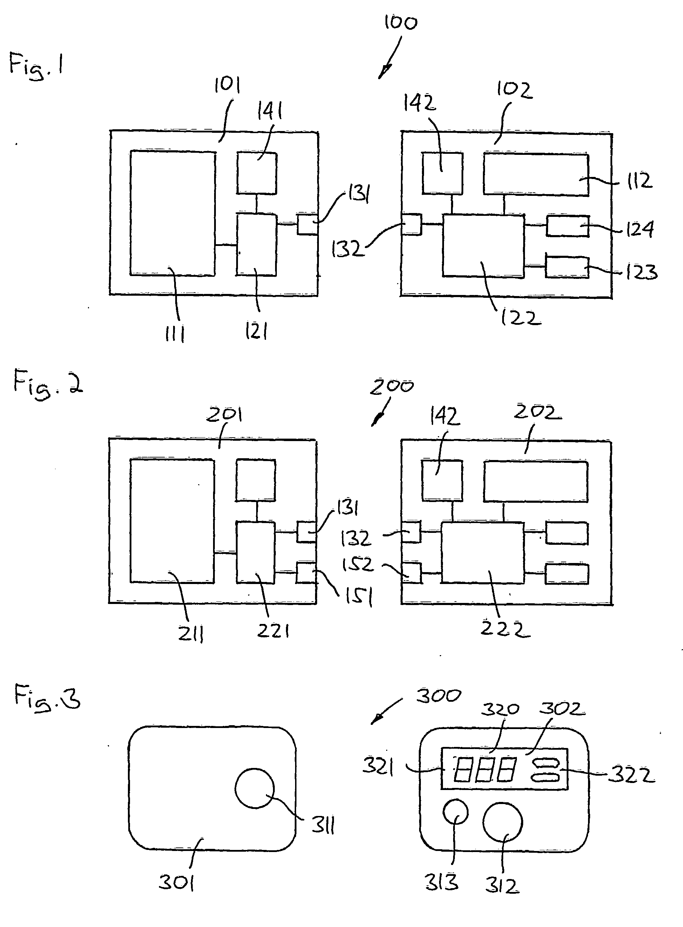

Fig. 1 shows a schematic representation of a first embodiment of the invention. Correspondingly, the configuration of the different structures as Well as there relative dimensions are intended to serve illustrative purposes only.

More specifically, a drug delivery system (here: infusion system) 100 comprises an infusion device 101 and an indicating device 102. The infusion device comprises an infusion unit 111 having a drug reservoir and a pump adapted for infusing a drug into a body of a user in accordance with instructions received from a first processor 121. The pump may be of the metering type, i.e. the amount of drug infused corresponds to the controlling signals received from the processor or the infusion unit may be provided with detecting means for determining the amount of drug actually infused. The first processor is associated with a transmitter 131 for transmitting data information (in the following also just data) to the indicating device. The data comprises information as to initial amount of drug in the reservoir, the current amount of drug in the reservoir, the infusion rate and ID information identifying the given infusion device. An energy source 141 is provided in the form of a battery.

The indicating device comprises a second processor 122 associated with a receiver 132 for receiving data from the first processor, as well as a timer means 123 and memory means 124 for storing the received data. The second processor is adapted for calculating an estimated time-dependent value based upon the received data and time information, and is associated With display means 112 for indicating a calculated value. The timer means will typically be a quartz controlled clock device provided integrally with the processor in the form of a single integrated circuit (IC) which also comprises ROM memory for storing an executing program instructions as well as RAM memory for storing the received data information. An energy source 142 is provided in the form of a battery.

The first embodiment is based on one-way transmission of information from the infusion device to the indicating device. The data may be transmitted automatically at preset intervals or upon manual actuation of the infusion device.

Fig. 2 shows a schematic representation of a second embodiment of the invention, in which like numerals are used to identify like structures. The second embodiment corresponds generally to the first embodiment, however, the drug infusion system 201 comprises means providing two-way communication between the indication device and the infusion device.

More specifically, the indication device comprises a second transmitter 152 associated with the second processor for transmitting commands to the indicating device. Correspondingly, the infusion device comprises a second receiver 151 associated with the first processor for receiving the commands.

The two-way communication may be utilized to make the indicating device the "controlling" unit. In this way every transmission of data from the infusion device is initialized with a request command for transmitting data representing the actual state of the infusion device. In addition to the request command also the initially received ID data may be resend for control purposes. Further, bolus commands may be send to the infusion device.

Fig. 3 shows a third embodiment of an infusion system 300 comprising means for two-way communication as described with reference to fig. 2. The system comprises an infusion device in the form of a skin-mountable pump unit and a combined indication and command device in the form of a portable "remote" unit.

In the shown embodiment the pump is a prefilled disposable unit 301 comprising a single actuation button 311. The remote unit comprises a display 320 as well as first and second buttons 312, 313, and may be adapted to be worn as a wristwatch, as a key-hanger or simply in a pocket. The display comprises a first portion 321 for indicating an amount of a drug (e.g. a number of insulin units IU) and a second portion 322 for status and alarm indications.

In a situation of use the pump device is mounted on a skin surface of the user and the start button is actuated. This may either result in data being transmitted instantly to the remote unit, however, in an exemplary embodiment actuation of the start button brings the pump in a "ready to be started" state awaiting a start command from the remote unit, however, should this not happen within a predefined period of time, the pump device may be adapted to start after this period. To transmit such a start command the command button 312 is actuated for a certain time (e.g. 5 seconds) after which initializing data is transmitted back to the remote, these data comprising e.g. pump unit ID, amount of drug contained in the reservoir and a preset basal infusion rate, however, the latter may also be set by the remote. After this procedure the system is "up and running" with the remote displaying estimated (or actual) values for the amount of drug infused or left in the reservoir.

Depending on the type of the transmission means, different update activities will take place. In case medium range transmission means (e.g. RF) is used, the remote unit may call for updates at regular intervals, e.g. every 5 minutes, whereby the remote unit quasi would display non-calculated status information. In case no data is transferred (e.g. the distance is too large between the two units) the displayed values will be based on estimates and after a certain preset period the display will indicate that it is time to update information. This may be done by bringing the two units within reach of each other and thereafter actuate the command button 312. In case short range transmission means (e.g. IR or induction) is used, the two units are brought within reach of each other and the command button 312 is actuated to command an update of the data information. Between updates the displayed values will be based on estimates. If desired the display may indicate whether the shown value is actual or a calculated estimate. After a certain preset period the display will indicate that it is time to update information again.

The remote unit 302 also comprises a bolus button allowing the user to infuse an additional amount of drug, for example of insulin at mealtime. When pressing the bolus button (e.g. one time for each IU) the display shows the corresponding amount. By actuating the command button 312 a combined bolus and update command is transmitted to the pump unit which will confirm that the bolus command has been received by transmitting updated data information together with confirm data indicating that the bolus command has been received and will be executed. The updated data representing the amount of current drug in the reservoir will have been adjusted corresponding to the bolus. The buttons may also be used to toggle the display between different display states, e.g. showing an infused or remaining amount of drug.

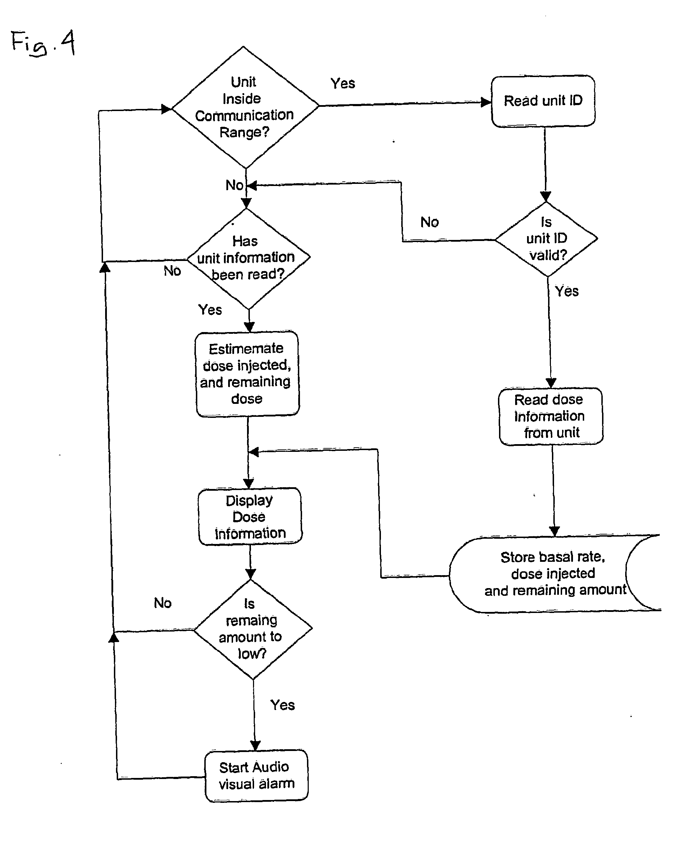

Fig. 4 shows a flow chart for a system corresponding to the third embodiment. More specifically, the system is based upon medium range transmitting means allowing automatic update commands to be sent from the remote unit. The flow chart illustrates a situation of use after the initialization/start procedure has been completed.

If the units are within communication range, the update command will result in data transmission from the pump unit comprising a pump unit ID which will be read by the indicating device. If the unit ID corresponds to the initially received ID then the update data is read and transferred to the memory from where it can be displayed as a non-estimated value. If the remaining amount of drug in the pump reservoir is below a given level a combined audiovisual alarm is activated. If the units are not within communication range, estimated values for the amount of drug infused or remaining in the pump reservoir will be calculated and displayed based on previously received information. An alarm may also indicate that no update information has been received within a preset time period. In case means for de-activating an alarm function is provided, then the alarm function should be re-activated automatically after a preset period.

As mentioned above, the units of the system may be supplied independently of each other which allows for a very flexible system. For example, a disposable pump unit can be used as a stand-alone unit which is mounted on the skin and started with a button. The disposable unit may work with a fixed default dose rate or may be programmed with a desired rate or profile using a remote unit. The system has a flexible architecture making it possible to expand the system into a product family by varying one or both of the members. For example, the pump unit may be provided as a disposable unit offering a range of different drug volumes, a range of different adhesives, or a range of different basal rates. The remote unit may vary from a simple reservoir estimator and display unit, to a top-of-the-line unit with advanced programming facilities, e.g. bolus, change of basal rate, profiles, history log etc. The remote unit may be provided with additional input and output means allowing it to communicate with external computer- and/or expert systems, just as the remote unit may be used in combination with a closed loop system comprising a blood glucose sensor. The remote unit may also be incorporated into a more general "platform" such as a PDA, handheld computer, cellular phone or wristwatch.

In the above description of exemplary embodiments, the different structures providing the desired relations between the different components just as the means providing the described functionality for the different components (processor means, transmitting and receiving means, memory and timer means) have been described to a degree to which the concept of the present invention will be apparent to the skilled reader. The detailed construction and specification for the different structures are considered the object of a normal design procedure performed by the skilled person along the lines set out in the present specification.

## Claims

1. A drug delivery system (100) comprising a delivery device (101) and an indicating device (102), the delivery device comprising:
- a reservoir (111) containing an amount of a liquid drug,
- means (111, 121) for delivering the drug into a body of a user in accordance with a delivery rate value or profile,
- first processor means (121), and
- first transmission means (131) cooperating with the first processor means for transmitting data information to receiving means in the indicating device,
the indicating device comprising:
- first receiving means (132) for receiving data information transmitted from the delivery device,
- memory means (124) for storing data information,
- timer means (123), **characterized by**
- second processor means (122) adapted for calculating an estimated time-dependent value based upon received data information transmitted from the delivery device and time information from the timer means, and
- indication means (112) cooperating with the second processor means for indicating a calculated value.

2. A drug delivery system as defined in claim 1, wherein the first processor means is adapted for:
- transmitting data information representing a current amount of drug contained in the reservoir, the second processor means being adapted for:
- storing data information representing the current amount of a drug,
- calculating a time-dependent estimate for the drug on the basis of the current amount, the time information, and a delivery rate value or profile stored in the memory means.

3. A drug delivery system as defined in claim 1, wherein the first processor means is adapted for:
- transmitting data information representing an initial amount of drug contained in the reservoir, a current amount of the drug and a delivery rate value or profile for the drug, the second processor means being adapted for
- storing the received data information,
- calculating a time-dependent estimate for the drug on the basis of the current amount, the time information, and the delivery rate value or profile.

4. A drug delivery system as defined in claim 3, wherein the second processor means is adapted for calculating at least one of the following values: the amount of drug delivered, the amount of drug remaining in the reservoir.

5. A drug delivery system as defined in any of the previous claims, wherein the second processor means is adapted for:
- determining the time lapsed since the last data information was received, and
- indicating information indicative of the time lapsed.

6. A drug delivery system as defined in any of the previous claims, wherein the delivery device further comprises:
- second receiving means (151) for receiving commands transmitted from the indicator device, the indicator device further comprising:
- second transmission means (152) for transmitting commands to the delivery device, the second transmission means being controllable by the user and/or by the second processor means.

7. A drug delivery system as defined in claim 6, wherein the first transmission means is operated in response to commands received from the second transmission means.

8. A drug delivery system as defined in claim 6, wherein the first transmission means is operated in response to commands received from the second transmission means operated by the second processor means at predetermined intervals.

9. A drug delivery system as defined in any of claims 6-8, wherein the indicating device further comprises means (312, 313) for inputting a bolus command, the second processor means being adapted for transmitting the bolus command to the delivery device, wherein the delivery device is adapted for delivering a bolus in response to the bolus command and for transmitting data information to the indicating device confirming that the bolus command has been received, and wherein the second processor means is adapted for calculating the time-dependent estimate for the drug utilizing the additional data information for the bolus command.

10. A drug delivery system as defined in any of the previous claims, wherein the first processor means is adapted to transmit ID data information indicative of a unique delivery device, the second processor means being adapted for checking the ID data information before storing updated data information in the memory means.

11. A drug delivery system as defined in any of the previous claims, wherein the data information stored in the memory means can be indicated by the indication means.

12. A drug delivery system as defined in any of the previous claims, wherein the indication means is in the form of a display (320) allowing continuous display of an estimated value.

13. An indicating device (102, 202, 302), comprising:
- receiving means (132) for receiving a signal containing data information,
- memory means (1, 24) for storing data information,
- timer means (123), **characterised by**
- processor means (122), where the processor means is adapted for calculating an estimated time-dependent value based upon received data information and time information received from the timer means, and
- indication means (112) for indicating a calculated value.

14. An indicating device as defined in claim 13, wherein the receiving means is adapted for receiving a remotely generated signal containing data information.

15. An indicating device as defined in claim 13, comprising the features of the indicating device as defined in any of the previous claims.

16. An indicating device as defined in claim 13, in combination with a delivery device comprising:
- a reservoir containing an amount of a liquid drug,
- means for delivering the drug into a body of a user in accordance with a delivery rate value or profile,
- means for providing data information to the indicating device, the means being readable or actuatable by the indicating device.

## Patentansprüche

1. Arzneistoffabgabesystem (100), umfassend eine Abgabevorrichtung (101) und eine Anzeigevorrichtung (102), wobei die Abgabevorrichtung umfasst:
- einen eine Menge eines flüssigen Arzneistoffs enthaltenden Behälter (111),
- ein Mittel (111, 121) zum Abgeben des Arzneistoffs in den Körper eines Anwenders gemäß eines Abgabegeschwindigkeitswerts oder - profils,
- ein erstes Verarbeitungsmittel (121) und
- ein erstes Übertragungsmittel (131), das mit dem ersten Verarbeitungsmittel zusammenarbeitet, um Dateninformation an ein Empfangsmittel in der Anzeigevorrichtung zu übertragen, wobei die Anzeigevorrichtung umfasst:
- ein erstes Empfangsmittel (132) zum Empfangen von Dateninformation, die von der Abgabevorrichtung übertragen wurde,
- ein Speichermittel (124) zum Speichern von Dateninformation,
- ein Timermittel (123), **gekennzeichnet durch**
- ein zweites Verarbeitungsmittel (122), das angepasst ist zum Berechnen eines geschätzten zeitabhängigen Werts auf der Basis von empfangener Dateninformation, die von der Abgabevorrichtung übertragen wurde, und Zeitinformation von dem Timermittel, und
- ein Anzeigemittel (112), das mit dem zweiten Verarbeitungsmittel zum Anzeigen eines berechneten Werts zusammenarbeitet.

2. Arzneistoffabgabesystem wie in Anspruch 1 definiert, wobei das erste Verarbeitungsmittel angepasst ist zum:
- Übertragen von Dateninformation, die eine aktuelle Menge an in dem Behälter enthaltenem Arzneistoff darstellt, wobei das zweite Verarbeitungsmittel angepasst ist zum:
- Speichern von die aktuelle Arzneistoffmenge darstellender Dateninformation,
- Berechnen einer zeitabhängigen Schätzung für den Arzneistoff auf der Basis der aktuellen Menge, der Zeitinformation und eines im Speichermittel gespeicherten Abgabegeschwindigkeitswerts oder - profils.

3. Arzneistoffabgabesystem wie in Anspruch 1 definiert, wobei das erste Verarbeitungsmittel angepasst ist zum:
- Übertragen von Dateninformation, die eine anfängliche Menge von im Behälter enthaltenem Arzneistoff, eine aktuelle Menge des Arzneistoffs und ein/en Abgabegeschwindigkeitswert oder -profil für den Arzneistoff darstellt, wobei das zweite Verarbeitungsmittel angepasst ist zum:
- Speichern der empfangenen Dateninformation,
- Berechnen einer zeitabhängigen Schätzung für den Arzneistoff auf der Basis der aktuellen Menge, der Zeitinformation und des Abgabegeschwindigkeitswerts oder -profils.

4. Arzneistoffabgabesystem wie in Anspruch 3 definiert, wobei das zweite Verarbeitungsmittel angepasst ist zum Berechnen von mindestens einem der folgenden Werte: der Menge an abgegebenem Arzneistoff, der Menge an im Behälter verbliebenem Arzneistoff.

5. Arzneistoffabgabesystem wie in einem der vorangehenden Ansprüche definiert, wobei das zweite Verarbeitungsmittel angepasst ist zum:
- Bestimmen der Zeit, die vergangen ist, seitdem die letzte Dateninformation empfangen wurde, und
- Anzeigen von Information, die für die vergangene Zeit hinweisend ist.

6. Arzneistoffabgabesystem wie in einem der vorangehenden Ansprüche definiert, wobei die Vorrichtung ferner umfasst:
- ein zweites Empfangsmittel (151) zum Empfangen von Befehlen, die von der Anzeigevorrichtung übertragen wurden, wobei die Anzeigevorrichtung ferner umfasst:
- ein zweites Übertragungsmittel (152) zum Übertragen von Befehlen an die Abgabevorrichtung, wobei das zweite Übertragungsmittel durch den Anwender und/oder durch das zweite Verarbeitungsmittel regelbar ist.

7. Arzneistoffabgabesystem wie in Anspruch 6 definiert, wobei das erste Übertragungsmittel als Antwort auf von dem zweiten Übertragungsmittel empfangenen Befehlen betrieben wird.

8. Arzneistoffabgabesystem wie in Anspruch 6 definiert, wobei das erste Übertragungsmittel als Antwort auf Befehle betrieben wird, die von dem durch das zweite Verarbeitungsmittel betriebenen zweiten Übertragungsmittel in vorbestimmten Zeitabständen empfangen werden.

9. Arzneistoffabgabesystem wie in einem der Ansprüche 6-8 definiert, wobei die Anzeigevorrichtung ferner Mittel (312, 313) zum Eingeben eines Bolusbefehls umfasst, wobei das zweite Verarbeitungsmittel angepasst ist zum Übertragen des Bolusbefehls an die Abgabevorrichtung, wobei die Abgabevorrichtung angepasst ist zum Abgeben eines Bolus' als Antwort auf den Bolusbefehl und zum Übertragen von Dateninformation an die Anzeigevorrichtung, wobei bestätigt wird, dass der Bolusbefehl empfangen wurde, und wobei das zweite Verarbeitungsmittel angepasst ist zum Berechnen der zeitabhängigen Schätzung für den Arzneistoff unter Verwendung der zusätzlichen Dateninformation für den Bolusbefehl.

10. Arzneistoffabgabesystem wie in einem der vorangehenden Ansprüche definiert, wobei das erste Verarbeitungsmittel angepasst ist zum Übertragen von ID-Dateninformation, die für eine besondere Abgabevorrichtung hinweisend ist, wobei das zweite Verarbeitungsmittel angepasst ist zum Überprüfen der ID-Dateninformation vor dem Speichern von aktualisierter Dateninformation im Speichermittel.

11. Arzneistoffabgabesystem wie in einem der vorangehenden Ansprüche definiert, wobei die im Speichermittel gespeicherte Dateninformation durch das Anzeigemittel angezeigt werden kann.

12. Arzneistoffabgabesystem wie in einem der vorangehenden Ansprüche definiert, wobei das Anzeigemittel in der Form eines Displays (320) vorliegt, das eine kontinuierliche Anzeige eines geschätzten Werts erlaubt.

13. Anzeigevorrichtung (102, 202, 302), umfassend:
- ein Empfangsmittel (132) zum Empfangen eines Dateninformation enthaltenden Signals,
- ein Speichermittel (124) zum Speichern von Dateninformation,
- ein Timermittel (123), **gekennzeichnet durch**
- ein Verarbeitungsmittel (122), wobei das Verarbeitungsmittel angepasst ist zum Berechnen eines geschätzten zeitabhängigen Werts auf der Basis von empfangener Dateninformation und von dem Timermittel empfangener Zeitinformation und
- ein Anzeigemittel (112) zum Anzeigen eines berechneten Werts.

14. Anzeigevorrichtung wie in Anspruch 13 definiert, wobei das Empfangsmittel angepasst ist zum Empfangen eines Dateninformation enthaltenden fernerzeugten Signals.

15. Anzeigevorrichtung wie in Anspruch 13 definiert, umfassend die Merkmale der wie in einem der vorangehenden Ansprüche definierten Anzeigevorrichtung.

16. Anzeigevorrichtung wie in Anspruch 13 definiert in Kombination mit einer Abgabevorrichtung, umfassend:
- einen eine Menge eines flüssigen Arzneistoffs enthaltenden Behälter,
- ein Mittel zum Abgeben des Arzneistoffs in den Körper eines Anwenders gemäß eines Abgabegeschwindigkeitswerts oder - profils,
- ein Mittel zum Bereitstellen von Dateninformation für die Anzeigevorrichtung, wobei das Mittel durch die Anzeigevorrichtung lesbar oder aktualisierbar ist.

## Revendications

1. Un système de délivrance de médicament (100) comprenant un dispositif de délivrance (101) et un dispositif indicateur (102), le dispositif de délivrance comprenant :
- un réservoir (111) contenant une quantité d'un médicament liquide,
- des moyens (111, 121) pour délivrer le médicament dans un corps d'un utilisateur en fonction d'une profil ou valeur de vitesse de délivrance,
- des premiers moyens processeurs (121) et
- des premiers moyens d'émission (131) coopérant avec les premiers moyens processeurs pour émettre des informations de données vers des moyens récepteurs dans le dispositif indicateur,
le dispositif indicateur comprenant :
- des premiers moyens récepteurs (132) pour recevoir des informations de données émises depuis le dispositif de délivrance,
- des moyens de mémoire pour mémoriser des informations de données,
- des moyens séquenceurs (123), **caractérisé par**
- des seconds moyens processeurs (122) aptes à calculer une valeur estimée dépendant du temps sur la base des informations de données reçues émises depuis le dispositif de délivrance et des informations de temps provenant des moyens séquenceurs, et
- des moyens indicateurs (112) coopérant avec les seconds moyens processeurs pour indiquer une valeur calculée.

2. Un système de délivrance de médicament selon la revendication 1, dans lequel les premiers moyens processeurs sont aptes à :
- émettre des informations de données représentant une quantité courante de médicament contenue dans le réservoir,
les seconds moyens processeurs étant aptes à :
- mémoriser des informations de données représentant la quantité courante d'un médicament,
- calculer une estimée fonction du temps pour le médicament sur la base de la quantité courante, des informations de temps, et d'un profil ou valeur de vitesse de délivrance mémorisé dans les moyens mémoire.

3. Un système de délivrance de médicament selon la revendication 1, dans lequel les premiers moyens processeurs sont aptes à :
- émettre des informations de données représentant une quantité initiale de médicament contenue dans le réservoir, une quantité courante du médicament et un profil ou valeur de vitesse de délivrance pour le médicament,
les seconds moyens processeurs étant aptes à :
- mémoriser les informations de données reçues,
- calculer une estimée dépendant du temps pour le médicament sur la base de la quantité courante, de l'information de temps, et du profil ou valeur de vitesse de délivrance.

4. Un système de délivrance de médicament selon la revendication 3, dans lequel les seconds moyens processeurs sont aptes à calculer au moins l'une des valeurs suivantes : la quantité de médicament délivrée, la quantité de médicament restant dans le réservoir.

5. Un système de délivrance de médicament selon l'une des revendications précédentes, dans lequel les seconds moyens processeurs sont aptes à :
- déterminer le temps écoulé depuis que les dernières informations de données ont été reçues, et
- indiquer des informations représentatives du temps écoulé.

6. Un système de délivrance de médicament selon l'une des revendications précédentes, dans lequel le dispositif de délivrance comprend en outre :
- des seconds moyens récepteurs (151) pour recevoir des ordres émis depuis le dispositif indicateur, le dispositif indicateur comprenant en outre :
- des seconds moyens d'émission (152) pour émettre des ordres vers le dispositif de délivrance, les seconds moyens d'émission étant contrôlables par l'utilisateur et /ou par les seconds moyens processeurs.

7. Un dispositif de délivrance de médicament selon la revendication 6, dans lequel les premiers moyens d'émission sont mis en oeuvre en réponse à des ordres reçus des seconds moyens d'émission.

8. Un système de délivrance de médicament selon la revendication 6, dans lequel les premiers moyens d'émission sont mis en oeuvre en réponse à des ordres reçus des seconds moyens d'émission mis en oeuvre par les seconds moyens processeurs à intervalles prédéterminés.

9. Un système de délivrance de médicament selon l'une des revendications 6 à 8, dans lequel le dispositif indicateur comprend en outre des moyens (312, 313) pour introduire un ordre de bolus, les seconds moyens processeurs étant aptes à émettre l'ordre de bolus vers le dispositif de délivrance, dans lequel le dispositif de délivrance est apte à délivrer un bolus en réponse à l'ordre de bolus et à émettre des informations de données vers le dispositif indicateur confirmant que l'ordre de bolus a été reçu, et dans lequel les seconds moyens processeurs sont aptes à calculer l'estimée fonction du temps pour le médicament en utilisant les informations de données additionnelles pour l'ordre de bolus.

10. Un dispositif de délivrance de médicament selon l'une des revendications précédentes, dans lequel les premiers moyens processeurs sont aptes à émettre des informations de données d'identification représentatives d'un dispositif de délivrance particulier, les seconds moyens processeurs étant aptes à vérifier les informations de données d'identification avant mémorisation des informations de données mises à jour dans les moyens mémoire.

11. Un système de délivrance de médicament selon l'une des revendications précédentes, dans lequel les informations de données mémorisées dans les moyens mémoire peuvent être indiquées par les moyens indicateurs.

12. Un système de délivrance de médicament selon l'une des revendications précédentes, dans lequel les moyens indicateurs sont sous forme d'un afficheur (320) autorisant un affichage continu d'une valeur estimée.

13. Un dispositif indicateur (102, 202, 302) comprenant :
- des moyens récepteurs (132) pour recevoir un signal contenant des informations de données,
- des moyens mémoire (1, 24) pour mémoriser des informations de données,
- des moyens séquenceurs (123), **caractérisé par**
- des moyens processeurs (122), où les moyens processeurs sont aptes à calculer une valeur estimée fonction du temps sur la base des informations de données reçues et d'informations de temps reçues des moyens séquenceurs, et
- des moyens indicateurs (142) pour indiquer une valeur calculée.

14. Un dispositif indicateur selon la revendication 13, dans lequel les moyens récepteurs sont aptes à recevoir un signal produit à distance contenant des informations de données.

15. Un dispositif indicateur selon la revendication 13, comprenant les caractéristiques du dispositif indicateur telles que définies dans l'une des revendications précédentes.

16. Un dispositif indicateur selon la revendication 13, en combinaison avec un dispositif de délivrance comprenant :
- un réservoir contenant une quantité d'un médicament liquide,
- des moyens pour délivrer le médicament dans un corps d'un utilisateur conformément à un profil ou valeur de vitesse de délivrance,
- des moyens pour délivrer des informations de données au dispositif indicateur, les moyens étant lisibles ou actionnables par le dispositif indicateur.
